# EUROPÄISCHE PATENTSCHRIFT

(11) **EP 2 585 131 B1**
(45) Veröffentlichungstag und Bekanntmachung des Hinweises auf die Patenterteilung: **16.09.2015**
(21) Anmeldenummer: 11733787.3
(22) Anmeldetag: 21.06.2011
(51) Int. Cl.: A61M 1/36

(54) **VORRICHTUNG ZUM DETEKTIEREN VON FEUCHTIGKEIT ZUR VERWENDUNG MIT EINER VORRICHTUNG ZUR ÜBERWACHUNG EINES ZUGANGS ZU EINEM PATIENTEN**
DEVICE FOR DETECTING MOISTURE FOR USE WITH A DEVICE FOR MONITORING ACCESS TO A PATIENT
DISPOSITIF DE DÉTECTION D'HUMIDITÉ DESTINÉ À ÊTRE UTILISÉ AVEC UN DISPOSITIF DE SURVEILLANCE DE L'ACCÈS À UN PATIENT

(30) Priorität: 22.06.2010 DE 102010024654
(43) Veröffentlichungstag der Anmeldung: 01.05.2013
(73) Patentinhaber: Fresenius Medical Care Deutschland GmbH, 61352 Bad Homburg v.d.H. (DE)
(72) Erfinder: HEPPE, John, 66606 St. Wendel (DE)
(74) Vertreter: Oppermann, Frank
(86) Internationale Anmeldenummer: PCT/EP2011/003044
(87) Internationale Veröffentlichungsnummer: WO 2011/160807

(56) Entgegenhaltungen:
- EP-A1- 2 550 038
- WO-A1-2006/008866
- WO-A1-2010/091852
- US-A1- 2002 198 483
- US-A1- 2005 038 325
- US-B1- 7 670 289

## Beschreibung

Die Erfindung bezieht sich auf eine Vorrichtung zum Detektieren von Feuchtigkeit zur Verwendung mit einer Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird. Darüber hinaus betrifft die Erfindung eine Vorrichtung zur Überwachung eines Zugangs zu einem Patienten, die über eine Vorrichtung zum Detektieren von Feuchtigkeit verfügt. Des weiteren betrifft die Erfindung eine Blutbehandlungsvorrichtung mit einem extrakorporalen Blutkreislauf, der eine arterielle Schlauchleitung mit einer arteriellen Kanüle und eine venöse Schlauchleitung mit einer venösen Kanüle aufweist, wobei die extrakorporale Blutbehandlungsvorrichtung über eine Vorrichtung zur Überwachung des arteriellen und/oder venösen Gefäßzugangs verfügt.

Auf dem Gebiet der Medizintechnik sind verschiedene Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicher zu stellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Trotz regelmäßiger Überwachung des Gefäßzugangs durch das Krankenhauspersonal besteht grundsätzlich die Gefahr, dass die Punktionskanüle unbemerkt aus dem Blutgefäß des Patienten herausrutscht. Die Gefahr des unbemerkten Herausrutschens der Punktionskanüle besteht auch bei der Heimdialyse. Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Es sind Vorrichtungen zur Überwachung eines Gefäßzugangs bekannt, die über eine Vorrichtung zum Detektieren von Feuchtigkeit verfügen, um an der Punktionsstelle das Austreten von Blut erkennen zu können. Die bekannten Vorrichtungen zum Detektieren von Feuchtigkeit, die bei den bekannten Überwachungsvorrichtungen für den Patientenzugang Verwendung finden, sind als auf die Punktionsstelle aufzulegendes Pad ausgebildet. Das Pad besteht aus einem saugfähigen Material, in das ein Feuchtigkeitssensor eingebettet ist.

Die WO 2006/008866 A1 und US 6,445,304 B1 beispielsweise beschreiben Vorrichtungen zum Detektieren von Feuchtigkeit aus einem saugfähigen Material, das auf die Haut aufgelegt. Die bekannten Pads zeichnen sich dadurch aus, dass der Feuchtigkeitssensor in das saugfähige Material eingebettet ist.

Der Feuchtigkeitssensor der WO 2006/008866 A1 weist zwei Leiterbahnen auf, die auf ein Trägermaterial aufgebracht sind. Die einen Enden beider Leiterbahnen sind als Anschlusskontakte ausgebildet, während die anderen Enden der Leiterbahnen mit einem Abschlusswiderstand elektrisch verbunden sind. Leiterbahnen und Abschlusswiderstand sind auf das Trägermaterial aufgedruckt. Hierzu wird eine leitfähige Tinte verwendet.

Aus der US 2005/038325 A1 ist eine Vorrichtung zum Detektieren von Feuchtigkeit bekannt, die zur Überwachung des Gefäßzugangs bei einer extrakorporalen Blutbehandlung Verwendung findet. Die Vorrichtung weist ein flexibles aus mehreren Lagen bestehendes Material auf, in das eine erste Leiterbahn und eine zweite Leiterbahn eingebettet sind. Beide Leiterbahnen bestehen aus elektrisch leitenden Drähten. An den einen Enden der Drähte sind die elektrischen Anschlussleitungen der Vorrichtung zur Überwachung des Patientenzugangs angeschlossen. Die anderen Enden der Drähte sind über einen Abschlusswiderstand elektrisch verbunden, der eine Überprüfung des Feuchtigkeitssensors erlaubt. Der Abschlusswiderstand ist zwischen zwei Lagen in das flexible Material eingebettet.

Während sich die Leiterbahnen mit verhältnismäßig geringem fertigungstechnischen Aufwand auf das Trägermaterial aufdrucken lassen, erweist sich die Herstellung eines aufgedruckten Abschlusswiderstands mit einem definierten Widerstand, der innerhalb relativ enger Toleranzen liegen soll, als relativ problematisch. Es hat sich zum einen gezeigt, dass aufgedruckte Abschlusswiderstände relativ großen fertigungsbedingten Toleranzen unterworfen sein können. Zum anderen hat sich gezeigt, dass die Biegewechselfestigkeit von auf ein Trägermaterial aufgedruckten Abschlusswiderständen relativ gering sein kann. Dies ist insofern problematisch, als der Abschlusswiderstand bei Bewegungen des Patienten ständig auf Biegung beansprucht wird.

Das Aufdrucken der Leiterbahnen auf das Trägermaterial, beispielsweise einen Vlies, kann in einem ersten Druckvorgang mit einer silberhaltigen Paste erfolgen, die nach dem Druckprozess getrocknet wird. In einem zweiten Druckvorgang kann der Abschlusswiderstand mit einer hochohmigen Graphitpaste auf das Trägermaterial aufgedruckt werden. Dabei sind unterschiedliche Ausbildungen der Leiterbahnenden und des Abschlusswiderstandes möglich.

Bei einer ersten Ausführungsform wird der zwischen zwei parallel verlaufenden Leiterbahnenden liegende Zwischenraum des Trägermaterials mit einer hochohmigen Graphitpaste bedruckt. Dabei haben die parallelen Leiterbahnenden einen Abstand von ca. 1 mm. Bei dieser Ausführungsform hat sich gezeigt, dass bereits eine Abweichung des Abstands der Leiterbahnenden um + - 10 % eine Abweichung des Widerstandswerts des Abschlusswiderstands um + - 50 % zur Folge haben kann. Solche Abweichungen des Abstands der Leiterbahnenden im Bereich des Abschlusswiderstandes können aufgrund von Schrumpfungsprozessen beim Trocken oder auch durch mechanische Beanspruchungen des Trägermaterials im praktischen Gebrauch beispielsweise bei Bewegungen des Patienten oder beim Abziehen einer Schutzfolie von dem Trägermaterial auftreten. Ein weiterer Nachteil dieser Ausführungsform liegt darin, dass ein auf dem Abschlusswiderstand selbst befindlicher kleiner Flüssigkeitstropfen nicht erkannt werden kann.

Bei einer alternativen Ausführungsform ist der Abschlusswiderstand nicht auf den zwischen zwei parallelen Leiterbahnenden liegenden Zwischenraum aufgedruckt, sondern der mit einer hochohmigen Graphitpaste auf das Trägermaterial aufgedruckte Abschlusswiderstand hat die Form eines U, dessen beide Schenkel an die beiden Leiterbahnenden angeschlossen sind, d.h. die beiden Leiterbahnenden setzten sich in unveränderter Breite in den Schenkeln des U-förmigen Abschlusswiderstandes fort. Dabei überlappen die Enden der Schenkel des U-förmigen Abschlusswiderstandes die beiden Leiterbahnenden. Bei dieser Ausführungsform hat sich gezeigt, dass die Abweichung des Widerstandswertes des Abschlusswiderstandes auf + - 30 % verringert werden kann, wobei es auf die Einhaltung eines bestimmten Abstands der Leiterbahnenden nicht unmittelbar ankommt.

Auch wenn sich mit der alternativen Ausführungsform geringere Toleranzen des Abschlusswiderstandes erreichen lassen, zeigt sich, dass die Erzielung eines reproduzierbaren Abschlusswiderstandes, der das Trägermaterial aufgedruckt wird, in der Praxis problematisch ist.

Ein weiterer Nachteil aufgedruckter Abschlusswiderstände liegt darin, dass Schweiß des Patienten den Widerstandswert des Abschlusswiderstands verringern kann. Des weiteren werden an die Biokompatibilität des aufgedruckten Abschlusswiderstands höhere Anforderungen gestellt, da der Abschlusswiderstand mit der Haut des Patienten in Kontakt kommen kann.

Ein weiterer Nachteil eines aufgedruckten Abschlusswiderstandes ergibt sich aus der Forderung, eine möglichst große Anzahl von Feuchtigkeitssensoren auf einem Druckbogen anzuordnen. Die Größe der Druckbögen (Vliesbögen), die in die Druckmaschine eingelegt werden können, ist aufgrund der Genauigkeitsanforderungen des Drucks begrenzt, wenn die Abschlusswiderstände nach dem Trocknen der in dem ersten Druckvorgang mit der silberhaltigen Paste aufgedruckten Leiterbahnen in dem zweiten Druckvorgang mit der Graphitpaste auf dem selben Druckbogen positionsgenau neben den Leiterbahnen aufgedruckt werden sollen. Aufgrund der Schrumpfung des Bogens beim Trocknen des ersten Drucks ergibt sich ein uneinheitlicher Versatz des zweiten Drucks gegenüber dem ersten Druck, der mit zunehmender Bogengröße ansteigt und ab einer gewissen Bogengröße nicht mehr toleriert werden kann, weil dann zumindest ein Teil der Abschlusswiderstände beim zweiten Druckvorgang nicht genau positioniert werden könnte. Aus Kostengründen ist jedoch die Verwendung möglichst großer Bögen mit möglichst vielen Einzelsensoren anzustreben, da die spezifischen Kosten eines einzelnen Sensors mit zunehmender Größe des Druckbogens abnehmen. Hierzu im Widerspruch steht der Wunsch, die besonders kostengünstig zu druckenden Feuchtigkeitssensoren mit einem Abschlusswiderstand einsetzen zu können.

Der Erfindung liegt die Aufgabe zugrunde, eine ohne großen fertigungstechnischen Aufwand herzustellende Vorrichtung zum Detektieren von Feuchtigkeit zur Verwendung mit einer Vorrichtung zur Überwachung eines Zugangs zu einem Patienten zu schaffen, die den mechanischen Beanspruchungen beispielsweise aufgrund von Bewegungen des Patienten sicher standhält, zu reproduzierbaren Messergebnissen führt und eine hohe Biokompatibilität hat.

Darüber hinaus liegt der Erfindung die Aufgabe zugrunde, eine Vorrichtung zur Überwachung eines Zugangs zu einem Patienten, insbesondere zur Überwachung des Gefäßzugangs bei einer extrakorporalen Blutbehandlung zu schaffen, die einen relativ geringen fertigungstechnischen Aufwand erfordert, den mechanischen Beanspruchungen beipielsweise aufgrund von Bewegungen des Patienten sicher standhält, zu reproduzierbaren Messergebnissen führt und eine hohe Biokompatibilität hat. Eine Aufgabe der Erfindung ist auch, eine extrakorporale Blutbehandlungsvorrichtung mit einer derartigen Vorrichtung zur Überwachung des Gefäßzugangs bereitzustellen.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung zum Detektieren von Feuchtigkeit ist als eine auf die Haut des Patienten aufzulegende Abdeckung (Pad) ausgebildet, die ein flexibles Trägermaterial aufweist, auf das als Feuchtigkeitssensor eine elektrisch leitende Struktur aus Leiterbahnen aufgebracht ist. Da die elektrisch leitende Struktur auf ein flexibles Trägermaterial aufgebracht, vorzugsweise aufgedruckt ist, ist die elektrisch leitende Struktur nicht Bestandteil des Trägermaterials.

Die Vorrichtung zum Detektieren von Feuchtigkeit zeichnet sich dadurch aus, dass die elektrisch leitende Struktur zwei Leiterbahnen aufweist, wobei das eine Ende der ersten Leiterbahn und das eine Ende der zweiten Leiterbahn ein erstes Paar von Anschlusskontakten und das andere Ende der ersten Leiterbahn und das andere Ende der zweiten Leiterbahn ein zweites Paar von Anschlusskontakten aufweist. Das erste Paar von Anschlusskontakten dient dem Anschluss der Vorrichtung zur Überwachung des Gefäßzugangs, während das zweite Paar von Anschlusskontakten dem Anschluss des Abschlusswiderstands dient. Da der Abschlusswiderstand nicht Bestandteil der Vorrichtung zum Detektieren von Feuchtigkeit ist, kann die Vorrichtung in großen Stückzahlen kostengünstig hergestellt werden.

Die Anschlusskontakte werden vorzugsweise von den Enden der Leiterbahnen gebildet, d.h. vorzugsweise sind die Enden oder Endabschnitte der Leiterbahnen als Anschlusskontakte ausgebildet. Dabei liegen die Anschlusskontakte vorzugsweise auf einer Seite des Pad.

Während die Vorrichtung zum Detektieren von Feuchtigkeit im Allgemeinen nur zur einmaligen Verwendung bestimmt ist, kann der Abschlusswiderstand als separates Bauteil nach einer gegebenenfalls erforderlichen Reinigung mehrfach wiederverwendet werden.

Da ein Abschlusswiderstand auf dem flexiblen Trägermaterial nicht vorhanden ist, sondern separat bereitgestellt wird, besteht nicht die Gefahr, dass der Abschlusswiderstand aufgrund von Bewegungen des Patienten zerstört werden könnte. Im Übrigen besteht auch nicht die Gefahr, dass sich der Widerstand des Abschlusswiderstands aufgrund von Biegewechselbeanspruchungen infolge der Bewegungen des Patienten ändern kann.

Da der Abschlusswiderstand in der Praxis nicht mit der Haut des Patienten in Berührung kommt, besteht nicht die Notwendigkeit den Abschlusswiderstand aus einem biokompatiblen Material herzustellen und nicht die Gefahr, dass Schweiß des Patienten den Widerstandswert des Abschlusswiderstandes verringern könnte.

Als Abschlusswiderstand kann ein konventioneller Widerstand Verwendung finden, der nicht auf das flexible Trägermaterial aufzubringen ist. Widerstände, die engen Toleranzen genügen, stehen kostengünstig zur Verfügung. Beispielsweise kann der Abschlusswiderstand ein SMD-Widerstand (Miniaturwiderstand) sein. Der Widerstandswert derartiger Widerstände hat im Allgemeinen eine Toleranz von nur +/- 1 %. Selbst wenn der SMD-Widerstand mit der Haut des Patienten in Berührung kommen sollte, hat der SMD-Widerstand eine bessere Biokompatibiltät im Vergleich zu einem aufgedruckten Widerstand.

Der Abschlusswiderstand ermöglicht mit den beiden Leiterbahnen die Überprüfung der Vorrichtung zum Detektieren von Feuchtigkeit auf ihre Funktionsfähigkeit durch eine Widerstandsmessung zwischen dem ersten Paar von Anschlusskontakten. Bei funktionsfähigem Feuchtigkeitssensor wird zwischen den Anschlusskontakten ein Widerstand gemessen, der der Summe des Abschlusswiderstands und der Widerstände beider Leiterbahnen entspricht.

Die Verwendung von separaten Abschlusswiderständen hat den Vorteil, dass auf einem Druckbogen eine große Anzahl von Einzelsensoren angeordnet werden können, weil die Schrumpfung des Druckbogens bei einem externen Abschlusswiderstand für eine genaue Positionierung des Widerstands ohne Bedeutung ist. Im Übrigen entfällt der Druckvorgang zum Aufbringen der Abschlusswiderstände, sodass beim Drucken Herstellungskosten eingespart werden.

Die Vorrichtung zum Detektieren von Feuchtigkeit kann in unterschiedlichen Formen ausgebildet sein. Sie kann nicht nur bei Blutbehandlungsvorrichtungen verwendet werden, die einen Gefäßzugang über eine Kanüle oder Nadel schaffen, sondern ist grundsätzlich auch zur Verwendung bei anderen Vorrichtungen geeignet. Folglich ist die erfindungsgemäße Vorrichtung zum Detektieren von Feuchtigkeit nicht auf die Verwendung bei Vorrichtungen zur Überwachung eines Zugangs zu einem Patienten beschränkt.

Ein weiteres mögliches Anwendungsgebiet der erfindungsgemäßen Vorrichtung zum Detektieren von Feuchtigkeit ist beispielsweise die Überwachung von Konnektoren in der Medizintechnik, beispielsweise Luer-Verbindungen oder Luer-Lock-Verbindungen. Derartige Konnektoren bestehen aus einem männlichen und einem weiblichen Verbindungsstück, die luft- und flüssigkeitsdicht miteinander verbunden werden können. Bei fehlerhaft verbundenen Konnektoren kann es zu Leckagen kommen. Luer-Konnektoren und Luer-Lock-Konnektoren finden sich beispielsweise am Schlauchsystem eines extrakorporalen Blutkreislaufs. Dort können die Konnektoren mit der erfindungsgemäßen Vorrichtung zur Erkennung eines Blutverlustes aufgrund einer Leckage überwacht werden.

Zur Überwachung von Leckagen an Luer- oder Luer-Lock-Konnektoren sollte die Vorrichtung zum Detektieren von Feuchtigkeit (Pad) besonders ausgebildet sein. Für diesen Anwendungsfall sollten die äußeren Abmessungen des Pads so bemessen sein, dass sich der oder die zu überwachenden Konnektoren mit dem Pad möglichst vollständig umwickeln lassen. Beim Umwickeln des Konnektors sollte die mit den Leiterbahnen bedruckte Seite des Sensors nach innen gerichtet sein, so dass aus dem Konnektor austretende Flüssigkeit, beispielsweise Blut, direkt auf die Leiterbahnen gelangen kann. An dem Pad und/oder dem Konnektor und/oder der mit dem Konvektor verbundenen Schlauchleitung können Mittel zum Befestigen des Pads an dem Konnektor und/oder der Schlauchleitung vorgesehen sein. Vorzugsweise sind Mittel zum Festkleben des Pads mit dem Konnektor und/oder der Schlauchleitung, beispielsweise Klebestreifen, vorgesehen.

Die beiden Leiterbahnen, die zu den Anschlusskontakten führen, können unterschiedlich ausgebildet sein. Sie können jeweils aus mehreren Abschnitten bestehen, die elektrisch miteinander verbunden sind. Allein entscheidend ist, dass die Enden der Leiterbahnen Anschlusskontakte aufweisen, um einerseits die Überwachungsvorrichtung und andererseits den Abschlusswiderstand anschließen zu können.

Die beiden Leiterbahnen können auf dem Trägermaterial grundsätzlich einen beliebigen Verlauf haben. Zur Erhöhung der Sensitivität sollten sie aber möglichst über ihre gesamte Länge nebeneinander liegend auf dem Trägermaterial angeordnet sein. Dabei sollte möglichst die gesamte auf dem Trägermaterial zu verfügende Fläche ausgenutzt werden.

Eine bevorzugte Ausführungsform sieht die Anordnung des ersten und zweiten Paars von Anschlusskontakten an einem Anschlusselement der Abdeckung vor. Damit sind sämtliche Anschlusskontakte von einer Seite leicht zugänglich. Vorzugsweise sind die Anschlusskontakte an dem Anschlusselement nebeneinander liegend angeordnet, so dass sie sich leicht kontaktieren lassen.

Bei einer weiteren bevorzugten Ausführungsform weisen die beiden Leiterbahnen jeweils zwei Abschnitte auf, wobei die miteinander zu verbindenden Enden beider Abschnitte jeder Leiterbahn jeweils an dem Anschlusselement der Abdeckung angeordnet sind. Diese Ausführungsform erlaubt die elektrische Verbindung der Leiterbahnabschnitte an dem Anschlusselement. Mit der Rückführung der Leiterbahnenden zu dem Anschlusselement lassen sich Kreuzungspunkte vermeiden, an denen sich die Leiterbahnen ansonsten kreuzen müssten, ohne aber dabei zu kontaktieren. Derartige Kreuzungspunkte sind bei gedruckten Leiterbahnen nicht oder nur mit großem Aufwand zu vermeiden.

Eine besonders bevorzugte Ausführungsform sieht vier Paare von Anschlusskontakten vor, wobei zwei Paare von Anschlusskontakten dem Anschluss der Vorrichtung zur Überwachung des Gefäßzugangs und des Abschlusswiderstands und zwei Paare von Anschlusskontakten zur elektrischen Verbindung der Leiterbahnabschnitte dienen.

Vorzugsweise sind die Leiterbahnen im Siebdruckverfahren auf das Trägermaterial aufgedruckt. Das Trägermaterial ist vorzugsweise ein Vlies, auf das sich die Leiterbahnen aufdrucken lassen. Die Oberfläche des Vliesstoffs sollte beispielsweise bei einer Druckfarbe auf Wasserbasis nicht hydrophob sein. Auch muss der Vliesstoff der für die Trocknung der Druckfarbe nötigen Belastung, beispielsweise der Wärme oder UV-Strahlung, standhalten können.

Die Abdeckung ist vorzugsweise U-förmig ausgebildet, kann aber auch reckförmig, rund oder oval sein. Die U-förmige Abdeckung erlaubt, die Kanüle für den Patientenzugang sowohl vor als auch nach dem Anbringen der Abdeckung auf der Haut des Patienten zu legen. Anstelle einer U-förmigen Abdeckung kann aber in der Abdeckung auch eine Ausnehmung zum Durchführen der Kanüle vorgesehen sein. Dann lässt sich die Kanüle aber erst nach dem Anbringen der Abdeckung legen.

Bei einer weiteren besonders bevorzugten Ausführungsform weist die Abdeckung einen oder mehrere Ausschnitte auf, die mit entsprechend ausgebildeten Ansätzen eines an die Abdeckung anzuschließenden Anschlussteils der Vorrichtung zur Überwachung des Patientenzugangs der Ausrichtung und Fixierung der Abdeckung dienen.

Die erfindungsgemäße Vorrichtung zur Überwachung eines Zugangs zu einem Patienten, insbesondere zur Überwachung des Gefäßzugangs bei einer extrakorporalen Blutbehandlung, verfügt über die erfindungsgemäße Vorrichtung zum Detektieren von Feuchtigkeit. Die Vorrichtung zur Überwachung des Patientenzugangs weist vorzugsweise eine an die Vorrichtung zum Detektieren von Feuchtigkeit anschließbare Auswerteinheit auf, die bei der Detektion von Feuchtigkeit einen akustischen und/oder optischen und/oder taktilen Alarm auslöst. Auch kann ein Steuersignal für einen Eingriff in die Steuerung der Einrichtung erzeugt werden, mit der über die Schlauchleitung eine Flüssigkeit dem Patienten zugeführt und/oder eine Flüssigkeit von dem Patienten abgeführt wird.

Die Überwachungsvorrichtung weist vorzugsweise einen Anschlussteil auf, an dem die Vorrichtung zum Detektieren von Feuchtigkeit angeschlossen wird, um eine elektrische Verbindung zwischen der Auswerteinheit der Überwachungsvorrichtung und dem Feuchtigkeitssensor der Vorrichtung zum Detektieren von Feuchtigkeit herzustellen. Der Anschlussteil der Überwachungsvorrichtung ist vorzugsweise über ein Verbindungskabel ausreichender Länge mit der Auswerteinheit elektrisch verbunden. Alternativ kann aber auch eine drahtlose Verbindung aufgebaut werden.

Bei einer bevorzugten Ausführungsform weist der Anschlussteil ein erstes und zweites Paar von Anschlusskontakten auf, an die das erste und zweite Paar von Anschlusskontakten der Vorrichtung zum Detektieren von Feuchtigkeit angeschlossen werden kann. An das erste Paar von Anschlusskontakten ist ein Verbindungskabel zur Herstellung einer elektrischen Verbindung zwischen der Auswerteinheit der Überwachungsvorrichtung und dem Feuchtigkeitssensor der Vorrichtung zum Detektieren von Feuchtigkeit angeschlossen, während das zweite Paar von Anschlusskontakten über einen Abschlusswiderstand elektrisch miteinander verbunden ist. Bei dieser Ausführungsform ist der Abschlusswiderstand Bestandteil des Anschlussteils des Verbindungskabels der Überwachungsvorrichtung. Es ist aber möglich, dass der Abschlusswiderstand nicht in dem Anschlussteil der Überwachungsvorrichtung, sondern in der Überwachungsvorrichtung, insbesondere in der Auswerteinheit, vorgesehen ist. Dann ist eine vieradrige Verbindungsleitung zu verwenden, wobei zwei Adern für den Feuchtigkeitssensor und zwei Adern für den Abschlusswiderstand bestimmt sind. Auch kann der Abschlusswiderstand an der Verbindungsleitung vorgesehen oder in die Verbindungsleitung integriert sein. Dabei kann der Abschlusswiderstand an jeder beliebigen Stelle der Verbindungsleitung angeschlossen sein. Eine weitere alternative Ausführungsform sieht ein Verbindungskabel vor, das einen Anschlussteil aufweist, mit dem das Verbindungskabel an die Überwachungsvorrichtung angeschlossen wird. Bei dieser alternativen Ausführungsform kann der Abschlusswiderstand Bestandteil des an die Überwachungsvorrichtung anzuschließenden Anschlussteils des Verbindungskabels sein.

Der Abschlusswiderstand ist vorzugsweise ein Miniaturwiderstand (SMD-Widerstand), der sich leicht in den Anschlussteil integrieren lässt. SMD-Widerstände haben geringe Bauteiltoleranzen und sind kostengünstig.

Die Reihenfolge der Anschlusskontakte an dem Anschlussteil ist beliebig. Es kommt nur darauf an, dass zwei Anschlusskontakte mit der Auswerteinheit und zwei Anschlusskontakte mit dem Abschlusswiderstand elektrisch verbunden werden können.

Der Anschlussteil der Überwachungsvorrichtung kann auch zwei weitere Paare von Anschlusskontakten aufweisen, mit denen sich die zugehörigen Anschlusskontakte der Vorrichtung zum Detektieren von Feuchtigkeit elektrisch verbinden lassen, die an den Enden der Leiterbahnabschnitte angeschlossen sind, um die elektrische Verbindung in dem Anschlussteil herzustellen. Die Enden der Leiterbahnabschnitte können aber auch direkt an dem Anschlusselement der Detektionsvorrichtung miteinander verbunden werden.

Der Anschlussteil der Überwachungsvorrichtung ist vorzugweise als Klemmvorrichtung zum Verklemmen der Abdeckung, insbesondere des Anschlusselements der Abdeckung ausgebildet. Die Klemmvorrichtung weist vorzugweise Mittel auf, mit denen die Vorrichtung zum Detektieren von Feuchtigkeit derart ausgerichtet und/oder fixiert wird, dass die Anschlusskontakte der Vorrichtung zum Detektieren von Feuchtigkeit den entsprechenden Anschlusskontakten des Anschlussteils der Überwachungsvorrichtung gegenüber liegen. Diese Mittel können als der Form der Vorrichtung zum Detektieren von Feuchtigkeit entsprechende Ausnehmungen oder der Form der Ausschnitte der Vorrichtung zum Detektieren von Feuchtigkeit entsprechende Ansätze ausgebildet sein. Die Fixierung kann durch Formschluss, Kraftschluss oder Reibschluss erfolgen. Als Mittel zum Fixieren können auch die Anschlusskontakte selbst ausgebildet sein. Beispielsweise können die Anschlusskontakte in die Abdeckung eindringende Dorne sein.

Die erfindungsgemäße Vorrichtung zur Überwachung eines Patientenzugangs kann eine separate Einheit bilden oder Bestandteil der Vorrichtung sein, mit der dem Patienten eine Flüssigkeit zugeführt und/oder von dem Patienten Flüssigkeit abgeführt wird, insbesondere Bestandteil der extrakorporalen Blutbehandlungsvorrichtung sein. Wenn die erfindungsgemäße Überwachungsvorrichtung Bestandteil der Blutbehandlungsvorrichtung ist, kann die Überwachungsvorrichtung von bestimmten Baugruppen oder Bauteilen Gebrauch machen, die in der Blutbehandlungsvorrichtung ohnehin vorhanden sind.

Im Folgenden wird ein Ausführungsbeispiel der Erfindung unter Bezugnahme auf die Zeichnungen näher erläutert.

Es zeigen:
- Fig. 1: die wesentlichen Komponenten einer Hämodialysevorrichtung, die über eine Vorrichtung zur Überwachung des arteriellen und venösen Gefäßzugangs verfügt,
- Fig. 2: ein Ausführungsbeispiel der Vorrichtung zum Detektieren von Feuchtigkeit der Vorrichtung zur Überwachung des arteriellen und venösen Gefäßzugangs in der Draufsicht,
- Fig.3: einen Schnitt durch die Vorrichtung zum Detektieren von Feuchtigkeit,
- Fig. 4: ein Ausführungsbeispiel des Anschlussteils der Vorrichtung zur Überwachung des Gefäßzugangs und
- Fig. 5: ein Ersatzschaltbild der Vorrichtung zur Überwachung des Gefäßzugangs und des Anschlussteils.

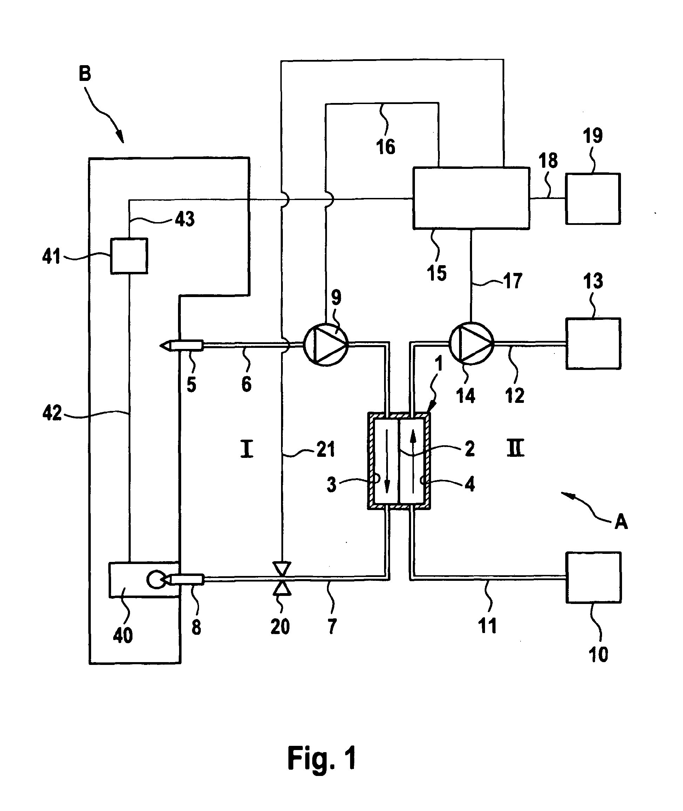

Fig. 1 zeigt die wesentlichen Komponenten einer Blutbehandlungsvorrichtung, insbesondere Hämodialysevorrichtung A, die über eine Vorrichtung B zur Überwachung des arteriellen und venösen Gefäßzugangs verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. An der Fistel oder dem Shunt des Patienten ist mittels einer arteriellen Punktionskanüle 5 eine arterielle Schlauchleitung 6 angeschlossen, die zu dem Einlass der Blutkammer 3 des Dialysators führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. In die arterielle Schlauchleitung 6 ist in eine Blutpumpe 9 geschaltet, die das Blut im extrakorporalen Blutkreislauf I fördert.

Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschlossen wird, wenn die venöse Punktionskanüle (Nadel) aus dem Gefäßzugang herausrutschen sollte und Feuchtigkeit (Blut) an der Punktionsstelle detektiert werden sollte. Darüber hinaus stoppt die Steuereinheit 15 nach dem Herausrutschen der Kanüle die Blutpumpe 9.

Die Überwachungsvorrichtung B dient bei dem vorliegenden Ausführungsbeispiel zur Überwachung des venösen Gefäßzugangs. Die Überwachungsvorrichtung B verfügt über eine Vorrichtung 40 zur Detektion von Feuchtigkeit, die an der Punktionsstelle angeordnet wird. Diese Detektionsvorrichtung 40 ist in Fig. 1 nur schematisch dargestellt. Darüber hinaus verfügt die Überwachungsvorrichtung über eine Auswerteinheit 41, die über eine Verbindungsleitung 42 mit der Detektionsvorrichtung 40 elektrisch verbunden ist.

Über eine Datenleitung 43 ist die Auswerteinheit 41 der Überwachungsvorrichtung B mit der zentralen Steuereinheit 15 der Dialysevorrichtung A verbunden. Für den Fall, dass Blut aus der venösen Kanüle und/oder der Punktionsstelle austritt und den Feuchtigkeitssensor befeuchtet, erzeugt die Auswerteinheit 41 ein Steuersignal, das die zentrale Steuereinheit 15 über die Datenleitung 43 empfängt, die einen Eingriff in die Blutbehandlung vornimmt. Die Steuereinheit 15 stoppt die Blutpumpe 9 und schließt die Schlauchklemme 20. Darüber hinaus erzeugt die Steuereinheit ein Alarmsignal, sodass die Alarmeinheit 19 einen akustischen und/oder optischen und/oder taktilen Alarm gibt. Die Daten können zwischen der Überwachungsvorrichtung B und der Dialysevorrichtung A auch drahtlos übertragen werden.

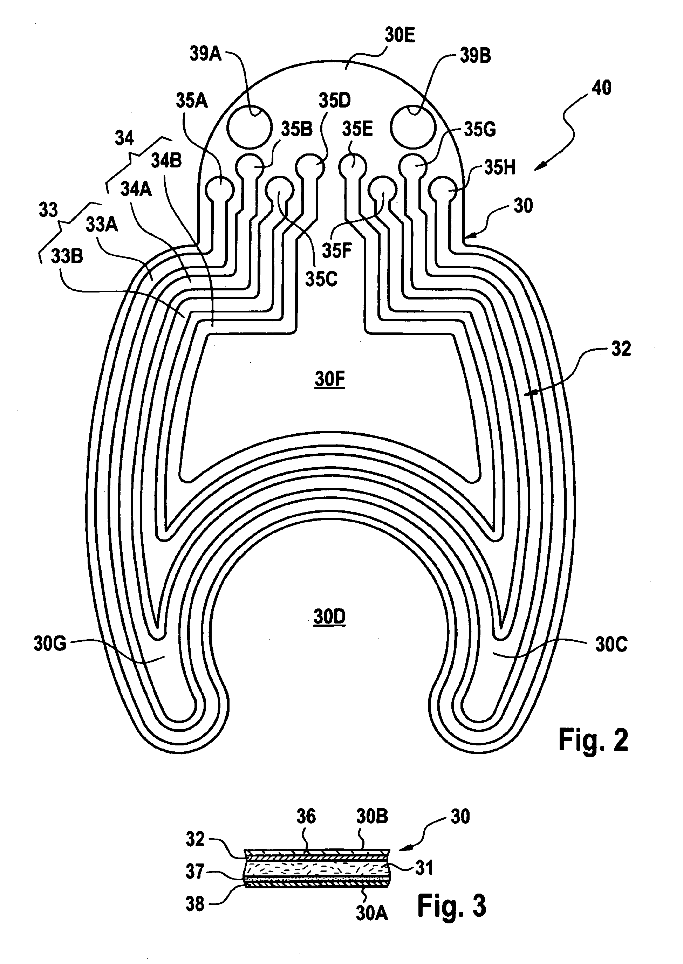

Nachfolgend wird unter Bezugnahme auf die Figuren 2 und 3 ein Ausführungsbeispiel der auf die Haut des Patienten an der Punktionsstelle aufzulegenden Vorrichtung 40 zum Detektieren von Feuchtigkeit beschrieben. Die Detektionsvorrichtung 40 ist als eine auf die Haut des Patienten aufzulegende Abdeckung (Pad) aus einem flexiblen Material ausgebildet. Das Pad 30 weist eine auf die Haut des Patienten aufzulegende Unterseite 30A und eine der Haut des Patienten abgewandte Oberseite 30B auf. Bei dem vorliegenden Ausführungsbeispiel ist das Pad 30 U-förmig ausgebildet. Das U-förmige Pad 30 weist einen mittleren Abschnitt 30F mit zwei Schenkeln 30G, 30C auf, die einen halbkreisförmigen Ausschnitt 30D seitlich umschließen. An dem mittleren Abschnitt 30F ist ein den beiden Schenkeln 30G, 30C gegenüberliegendes Anschlusselement 30E angeformt, das halbkreisförmig ausgebildet ist. Das Pad 30 besteht aus mehreren Schichten, die nachfolgend beschrieben werden (Fig. 3).

Das Pad 30 weist ein biokompatibles, hautverträgliches, weiches Trägermaterial 31 aus einem flexiblen Vliesstoff, bspw. aus einem Cellulose-/Polyester-Fasergemisch auf. Aufgrund seiner offenen Struktur ist der Vliesstoff sowohl für Luft als auch Wasserdampf durchlässig. Auf der Oberseite des Trägermaterials 31 befindet sich eine elektrisch leitende Struktur 32, die den eigentlichen Feuchtigkeitssensor bildet. Die elektrisch leitende Struktur des Feuchtigkeitssensors 32 weist eine erste Leiterbahn 33 und eine zweite Leiterbahn 34 auf. Die erste Leiterbahn 33 besteht aus zwei Abschnitten 33A und 33B. Auch die zweite Leiterbahn 34 besteht aus zwei Abschnitten 34A und 34B.

Der Feuchtigkeitssensor 32 weist acht Anschlusskontakte 35A - 35H auf, die auf der Oberseite des Anschlusselements 30E des Pads 30 angeordnet sind. Die Anschlusskontakte 35A - 35H sind auf dem Anschlusselement 30E im Wesentlichen nebeneinander liegend angeordnet. Dabei liegen die Anschlusskontakte 35B, 35D, 35E, 35G in einer ersten Reihe und die Anschlusskontakte 35A, 35C, 35F, 35H in einer zweiten Reihe, so dass die Anschlusskontakte gegeneinander leicht versetzt sind.

Der erste Leiterbahnabschnitt 33A der ersten Leiterbahn 33 erstreckt sich von dem ersten Anschlusskontakt 35A entlang des Randes des Pads 30 bis zu dem achten Anschlusskontakt 35H, während sich der erste Leiterbahnabschnitt 34A der zweiten Leiterbahn 34 von dem zweiten Anschlusskontakt 35B entlang des ersten Leiterbahnabschnitts 33A der ersten Leiterbahn zu dem siebten Anschlusskontakt 35G erstreckt. Von dem dritten Anschlusskontakt 35C erstreckt sich der zweite Leiterbahnabschnitt 33B der ersten Leiterbahn entlang des ersten Leiterbahnabschnitts 34A der zweiten Leiterbahn bis zu dem sechsten Anschlusskontakt 35F, während sich der zweite Leiterbahnabschnitt 34B der zweiten Leiterbahn 34 entlang des zweiten Leiterbahnabschnitts 33B der ersten Leiterbahn 33 von dem vierten Anschlusskontakt 35D zu dem fünften Anschlusskontakt 35E erstreckt. Die einzelnen Leiterbahnabschnitte 33A, 33B; 34A, 34B bilden damit jeweils eine nicht geschlossene Leiterschleife.

Die Leiterbahnen 33, 34 sowie die Anschlusskontakte 35 sind auf der Oberseite des Trägermaterials 31 aufgedruckt. Zum Bedrucken des Trägermaterials mit den Leiterbahnen und Kontaktelementen sind dem Fachmann geeignete Druckverfahren bekannt. Vorzugsweise werden die Leiterbahnen im Siebdruckverfahren auf das Trägermaterial aufgedruckt. Der Siebdruck erlaubt das Aufbringen relativ großer Schichtdicken, was für die Herstellung robuster Leiterbahnen auf dem Trägermaterial erforderlich ist. Vorzugsweise wird eine in einem thermoplastischen Harz gebundene Paste auf Silberbasis aufgebracht, wobei das thermoplastische Harz beim Aushärten unter Wärmeeinfluss evaporiert.

Auf der Oberseite des Trägermaterials 31 bzw. der Leiterbahnen 33, 34 befindet sich bei dem vorliegenden Ausführungsbeispiel eine feuchtigkeitsdurchlässige Deckschicht 36. Damit können Fehlalarme durch unbeabsichtigtes Berühren der Leiterbahnen vermieden werden. Die Deckschicht 36 erleichtert auch das Säubern des Pads, wenn es mit Flüssigkeiten in Berührung gekommen sein sollte. Auf diese Deckschicht kann aber auch verzichtet werden.

An der Unterseite ist das Trägermaterial 31 mit einer für Flüssigkeit undurchlässigen, aber für Dampf durchlässigen Adhäsionsschicht 37 versehen, die mit einer Abdeckschicht 38, insbesondere einem Silikonpapier kaschiert ist, das sich leicht von der Adhäsionsschicht lösen lässt. Vor dem Aufbringen des Pads 30 auf die Haut des Patienten wird das Silikonpapier 38 von der Adhäsionsschicht 37 abgezogen.

An dem Anschlusselement 30E befinden sich zwei runde Ausschnitte 39A, 39B zur Ausrichtung und Fixierung des Pads 30 an einem Anschlussteil 50 der Überwachungsvorrichtung B, der nachfolgend im Einzelnen beschrieben wird.

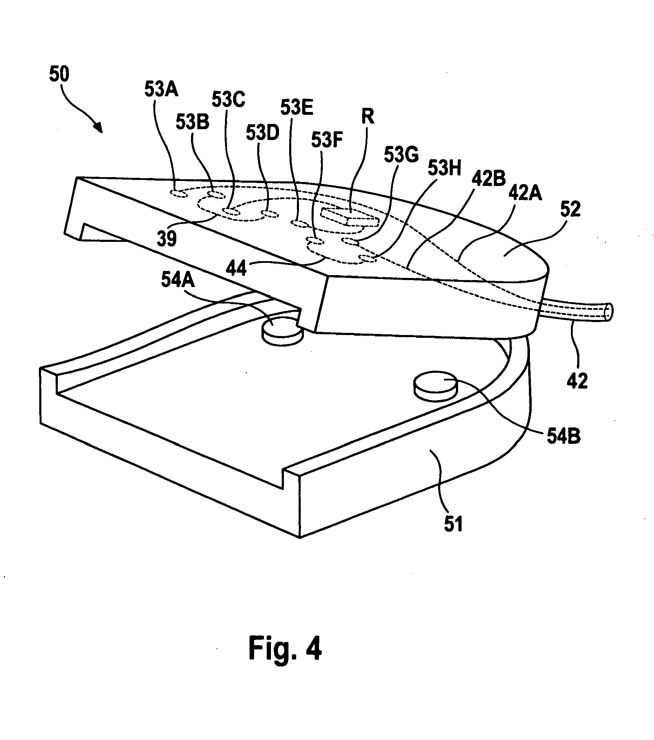

Fig. 4 zeigt ein Ausführungsbeispiel des Anschlussteils 50 der Überwachungsvorrichtung B zum Anschluss des Pads 30. Der Anschlussteil 50 ist als eine Klemmvorrichtung zum Verklemmen des Anschlusselements 30E des Pads 30 ausgebildet. Der Anschlussteil 50 weist einen unteren Klemmteil 51 und einen oberen Klemmteil 52 auf, wobei das Anschlusselement 30E des Pads passend in den unteren Klemmteil 51 eingelegt werden kann. In dem oberen Klemmteil 52 befinden sich den jeweiligen Anschlusskontakten 35A - 35H des Pads 30 zugeordnete Anschlusskontakte 53A - 53H, die an der Innenseite des oberen Klemmteils 52 nebeneinander liegend angeordnet sind. Zur Ausrichtung und Fixierung des Pads dienen zwei Ansätze 54A, 54B mit kreisförmigem Querschnitt an der Innenseite des unteren Klemmteils 51, die beim Einlegen des Anschlusselements 30E in die kreisförmigen Ausschnitte 39A, 39B greifen.

Wenn das Anschlusselement 30E des Pads 30 zwischen dem unteren und oberen Klemmteil 51, 52 des Anschlussteils 50 verklemmt ist, wird eine elektrische Verbindung zwischen den Kontaktelementen 35A - 35H des Pads 30 und den Kontaktelementen 53A - 53H des Anschlussteils 50 hergestellt.

An den ersten und siebten Anschlusskontakt 53A, 53G des Anschlussteils 50 sind die beiden Leiter 42A, 42B der Verbindungsleitung 42 angeschlossen, die zu der Auswerteinheit 41 der Überwachungsvorrichtung B führt. Der dritte und fünfte Anschlusskontakt 53C, 53E sind über einen Abschlusswiderstand R elektrisch miteinander verbunden. Der Abschlusswiderstand R kann ein in den oberen Klemmteil 52 integrierter SMD-Widerstand (Miniaturwiderstand) sein.

Der Anschlussteil 50 dient nicht nur zum Anschluss des Feuchtigkeitssensors 32 an die Auswerteinheit 41 der Überwachungsvorrichtung B und dem Anschluss des Abschlusswiderstands R, sondern auch der elektrischen Verbindung der beiden Leiterbahnabschnitte 33A, 33B; 34A, 34B der ersten und zweiten Leiterbahn 33, 34 des Feuchtigkeitssensors 32. Hierfür sind der zweite und vierte Anschlusskontakt 53B, 53D des Anschlussteils 50 mit einer ersten Brücke 39 und der sechste und achte Anschlusskontakt 53F, 53H über eine zweite Brücke 44 miteinander elektrisch verbunden. Dadurch wird eine elektrische Verbindung zwischen dem ersten und zweiten Leiterbahnabschnitt 33A, 33B der ersten Leiterbahn 33 bzw. dem ersten und zweiten Leiterbahnabschnitt 34A, 34B der zweiten Leiterbahn 34 hergestellt, wenn das Anschlusselement 30E des Pad 30 in dem Anschlussteil 50 verklemmt ist.

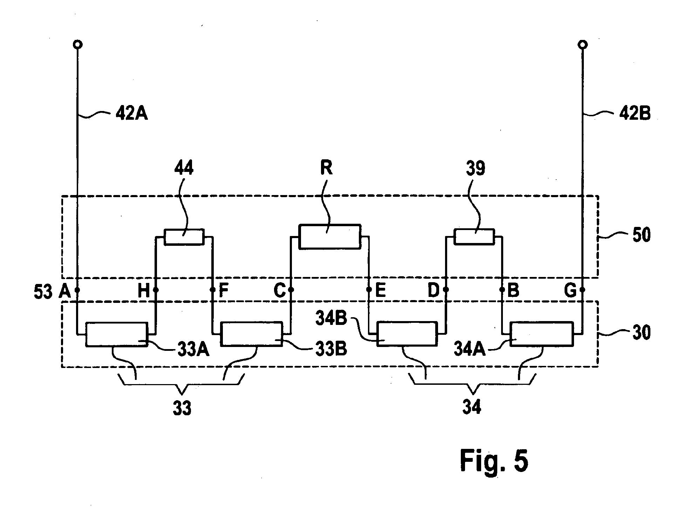

Mit der Brücke 44 werden die beiden Leiterbahnabschnitte 33A und 33B in Reihe geschaltet und stellen so die erste Leiterbahn 33 her. Analog werden mit der Brücke 39 die beiden Leiterbahnabschnitte 34A und 34B in Reihe geschaltet und stellen so die zweite Leiterbahn 34 her. Ein Ersatzschaltbild mit der Reihenschaltung der Leiterbahnabschnitte 33A und 33B bzw. 34A und 34B der Leiterbahnen 33 bzw. 34 zeigt Fig. 5.

Die Brücken 39 und 44 können auch an dem Anschlusselement 30E des Pads 30 vorgesehen sein, um die Anschlusskontakte 35B, 35D; 35F, 35H der Leiterbahnabschnitte 33A, 33B;34A, 34B miteinander zu verbinden. Die Brücken können auf das Anschlusselement 30E aufgedruckt sein oder elektrische Drähte sein. Für die Kontaktierung können die Anschlusskontakte auch entfallen, wobei die elektrische Verbindung direkt an den Leiterbahnenden vorgenommen werden kann. Bei dieser alternativen Ausführungsform können auch die entsprechenden Anschlusskontakte 53B, 53D; 53F, 53H an dem Anschlussteil 50 entfallen.

Der Gesamtwiderstand zwischen dem ersten und siebten Anschlusskontakt 53A, 53G des Anschlussteils 50, an die das zweiadrige Verbindungskabel 42 angeschlossen sind, das den Feuchtigkeitssensor 32 mit einer Auswerteinheit 41 der Überwachungsvorrichtung B elektrisch verbindet, setzt sich aus der Summe der Widerstände der beiden Leiterbahnen 33, 34 und des Abschlusswiderstands R zusammen. Bei dem Abschlusswiderstand R handelt es sich um einen hochohmigen Widerstand, insbesondere um einen Widerstand von größer als 100 kOhm (hochohmig), während die Leiterbahnwiderstände einen spezifischen Widerstand von kleiner als 5 Ohm/cm (niederohmig), beispielsweise 1 - 2 Ohm/cm aufweisen.

Die Auswerteinheit 41 der Überwachungsvorrichtung B misst den Widerstand zwischen den Anschlusskontakten 53A, 53G des Anschlussteils 50. Wenn das Pad 30 mit Flüssigkeit, insbesondere Blut benetzt werden sollte, verringert sich der zwischen den Anschlusskontakten gemessene Widerstand, so dass die Auswerteinheit 41 auf einen Blutaustritt infolge einer Nadeldiskonnektion schließt.

Die Auswerteinheit 41 erlaubt auch die Überprüfung der Funktionsfähigkeit des Feuchtigkeitssensors 32 der Detektionsvorrichtung 40. Hierzu misst die Auswerteinheit 41 den Widerstand zwischen den Anschlusskontakten 53A, 53G. Dieser Widerstand muss der Summe von Abschlusswiderstand R und Leiterbahnwiderständen entsprechen, wenn das Pad 30 nicht mit Flüssigkeit benetzt ist. Wenn der gemessene Widerstand um eine vorgegebene Differenz von dem Abschlusswiderstand abweichen sollte, stellt die Auswerteinheit 41 fest, dass der Feuchtigkeitssensor 32 nicht funktionsfähig ist.

Die Auswerteinheit kann einen Leiterbahnbruch beispielsweise aufgrund eines fehlerhaften Drucks bei der Herstellung oder eines zu hohen Stromflusses bei einer Überlastung des Stromkreises dadurch erkennen, dass die Auswerteinheit einen unendlich großen Widerstand (R → unendlich) zwischen den Auschlusskontakten feststellt, der auf einen nicht geschlossenen Stromkreis infolge des Leiterbahnbruchs zurückzuführen ist. Einen fehlerhaft angeschlossenen Feuchtigkeitssensor kann die Auswerteinheit ebenfalls durch einen unendlich großen Widerstand (R → unendlich) zwischen den Anschlusskontakten erkennen. Wenn sich die Leiterbahnen aufgrund eines fehlerhaften Drucks bei der Herstellung berühren sollten, kann dieser Fehler von der Auswerteinheit dadurch erkannt werden, dass ein unendlich kleiner Widerstand (R → Null) zwischen den Anschlusskontakten gemessen wird, der auf einen Kurzschluss zurückzuführen ist.

Die erfindungsgemäße Detektionsvorrichtung 40 mit den beiden Leiterbahnen 33, 34 hat den Vorteil, dass die besondere Leiterbahnführung die Verlagerung des Abschlusswiderstands R außerhalb des Pads erlaubt. Dadurch ist eine einfachere Fertigung des Pads möglich. Darüber hinaus ergibt sich der Vorteil einer verbesserten Stabilität gegenüber mechanischen Beanspruchungen und eines stets reproduzierbaren Abschlusswiderstands unabhängig von dem Fertigungsprozess des Pads.

Zum Gebrauch wird das Silikonpapier 38 von dem Pad 30 abgezogen und das Pad mit der Adhäsions- bzw. Klebeschicht 37 auf die Haut des Patienten aufgelegt und aufgeklebt. Anschließend kann die Punktion mit der Kanüle 8 erfolgen. Da das Pad 30 seitlich ausgeschnitten ist, ist es aber auch möglich, das Pad nach der Punktion auf die Haut des Patienten aufzulegen und aufzukleben. Es ist möglich, den Anschlussteil 50 vor oder nach dem Auflegen und Aufkleben des Pads 30 auf die Haut des Patienten das Pad anzuschließen.

## Patentansprüche

1. Vorrichtung zum Detektieren von Feuchtigkeit zur Verwendung mit einer Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird,
wobei die Vorrichtung zum Detektieren von Feuchtigkeit als eine auf die Haut des Patienten aufzulegende Abdeckung (30) ausgebildet ist, die ein flexibles Trägermaterial (31) aufweist, auf das als Feuchtigkeitssensor eine elektrisch leitende Struktur (32) aus Leiterbahnen (33,34) aufgebracht ist, die eine erste Leiterbahn (33) und eine zweite Leiterbahn (34) aufweist, wobei das eine Ende der ersten Leiterbahn und das eine Ende der zweiten Leiterbahn ein erstes Paar (35A, 35G) von auf das Trägermaterial (31) aufgebrachten Anschlusskontakten zum Anschluss der Vorrichtung zur Überwachung des Gefäßzugangs aufweist,
**dadurch gekennzeichnet, dass** das andere Ende der ersten Leiterbahn (33) und das andere Ende der zweiten Leiterbahn (34) ein zweites Paar (35C, 35E) von auf das Trägermaterial (31) aufgebrachten Anschlusskontakten zum Anschluss eines Abschlusswiderstandes aufweist, der nicht Bestandteil der Vorrichtung zum Detektieren von Feuchtigkeit ist.

2. Vorrichtung nach Anspruch 1, **dadurch gekennzeichnet, dass** das erste und zweite Paar von Anschlusskontakten (35A, 35G; 35C, 35E) an einem Anschlusselement (30E) der Abdeckung (30) angeordnet sind.

3. Vorrichtung nach Anspruch 2, **dadurch gekennzeichnet, dass** die Anschlusskontakte (35A, 35G; 35C, 35E) an dem Anschlusselement (30E) nebeneinander liegend angeordnet sind.

4. Vorrichtung nach Anspruch 2 oder 3, **dadurch gekennzeichnet, dass** die erste Leiterbahn (33) einen ersten Abschnitt (33A) und einen zweiten Abschnitt (33B) aufweist, wobei die beiden Enden des ersten Abschnitts und die beiden Enden des zweiten Abschnitts der ersten Leiterbahn an dem Anschlusselement (30E) der Abdeckung (30) angeordnet sind und
die zweite Leiterbahn (34) einen ersten Abschnitt (34B) und einen zweiten Abschnitt (34A) aufweist, wobei die beiden Enden des ersten Abschnitts und die beiden Enden des zweiten Abschnitts der zweiten Leiterbahn an dem Anschlusselement (30E) der Abdeckung (30) angeordnet sind.

5. Vorrichtung nach Anspruch 4, **dadurch gekennzeichnet, dass** das eine Ende des ersten Abschnitts der ersten Leiterbahn und das eine Ende des ersten Abschnitts der zweiten Leiterbahn die Anschlusskontakte (35A, 35G) des ersten Paars von Anschlusskontakten bilden und
das eine Ende des zweiten Abschnitts der ersten Leiterbahn und das eine Ende des zweiten Abschnitts der zweiten Leiterbahn die Anschlusskontakte (35C, 35E) des zweiten Paars von Anschlusskontakten bilden.

6. Vorrichtung nach Anspruch 5, **dadurch gekennzeichnet, dass** das andere Ende des ersten Abschnitts der ersten Leiterbahn und das andere Ende des zweiten Abschnitts der ersten Leiterbahn ein drittes Paar (35H, 35F) von Anschlusskontakten bilden und
das andere Ende des ersten Abschnitts der zweiten Leiterbahn und das andere Ende des zweiten Abschnitts der zweiten Leiterbahn ein viertes Paar (35B, 35D) von Anschlusskontakten bilden.

7. Vorrichtung nach einem der Ansprüche 1 bis 6, **dadurch gekennzeichnet, dass** die Abdeckung (30) einen oder mehrere Ausschnitte (39A, 39B) aufweist.

8. Vorrichtung nach einem der Ansprüche 1 bis 7, **dadurch gekennzeichnet, dass** die Abdeckung (30) U-förmig ausgebildet ist.

9. Vorrichtung nach einem der Ansprüche 1 bis 8, **dadurch gekennzeichnet, dass** das Trägermaterial (31) ein Vlies ist.

10. Vorrichtung nach einem der Ansprüche 1 bis 9, **dadurch gekennzeichnet, dass** die Leiterbahnen (33, 34) auf das Trägermaterial (31) im Siebdruckverfahren aufgedruckt sind.

11. Vorrichtung (B) zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, mit einer Vorrichtung zum Detektieren von Feuchtigkeit nach einem der Ansprüche 1 bis 10.

12. Vorrichtung nach Anspruch 11, **dadurch gekennzeichnet, dass** die Überwachungsvorrichtung (B) eine an die Vorrichtung zum Detektieren von Feuchtigkeit (40) anschließbare Auswerteinheit (41) aufweist.

13. Vorrichtung nach Anspruch 11 oder 12, **dadurch gekennzeichnet, dass** die Überwachungsvorrichtung (B) einen Anschlussteil (50) aufweist, an den die Vorrichtung (40) zum Detektieren von Feuchtigkeit anschließbar ist.

14. Vorrichtung nach Anspruch 13, **dadurch gekennzeichnet, dass** der Anschlussteil (50) ein erstes und zweites Paar (53A, 53G; 53C, 53E) von Anschlusskontakten zur Kontaktierung mit dem ersten und zweiten Paar (35A, 35G; 35C, 35E) von Anschlusskontakten der Vorrichtung (40) zum Detektieren von Feuchtigkeit aufweist, wobei das erste Paar (53A, 53G) von Anschlusskontakten an ein Verbindungskabel (42) zur Herstellung einer elektrischen Verbindung zwischen der Auswerteinheit (41) der Überwachungsvorrichtung (B) und der Vorrichtung zum Detektieren von Feuchtigkeit (40) angeschlossen sind, und das zweite Paar (53C, 53E) von Anschlusskontakten über einen Abschlusswiderstand (R) elektrisch miteinander verbunden sind.

15. Vorrichtung nach Anspruch 14, **dadurch gekennzeichnet, dass** der Abschlusswiderstand (R) ein SMD-Widerstand ist.

16. Vorrichtung nach Anspruch 14 oder 15, **dadurch gekennzeichnet, dass** der Anschlussteil (50) der Überwachungsvorrichtung (B) vier weitere Anschlusskontakte (53B, 53D; 53F, 53H) aufweist, von denen jeweils zwei Anschlusskontakte elektrisch miteinander verbunden sind.

17. Vorrichtung nach einem der Ansprüche 13 bis 16, **dadurch gekennzeichnet, dass** der Anschlussteil (50) der Überwachungsvorrichtung (B) als Klemmvorrichtung zum Verklemmen der Abdeckung (30) ausgebildet ist.

18. Blutbehandlungsvorrichtung mit einem extrakorporalen Blutkreislauf (I), der eine arterielle Blutleitung (6) mit einer arteriellen Kanüle (5) und eine venöse Blutleitung (7) mit einer venösen Kanüle (8) aufweist, und mit einer Vorrichtung zur Überwachung (B) des arteriellen und/oder venösen Gefäßzugangs nach einem der Ansprüche 11 bis 17.

## Claims

1. Device for detecting moisture for use with a device for monitoring an access to a patient for an apparatus with which a fluid is supplied to a patient or a fluid is carried away from the patient via a tube,
the device for detecting moisture being formed as a cover (30) which is to be applied to the patient's skin and has a flexible base material (31) to which an electrically conductive structure (32) made of conductor tracks (33, 34) is applied as a moisture sensor, which structure has a first conductor track (33) and a second conductor track (34), one end of the first conductor track and one end of the second conductor track having a first pair (35A, 35G) of connection contacts, which are applied to the base material (31), for connecting the device for monitoring the vascular access,
**characterised in that** the other end of the first conductor track (33) and the other end of the second conductor track (34) have a second pair (35C, 35E) of connection contacts, which are applied to the base material (31), for connecting a terminating resistor, which is not a component of the device for detecting moisture.

2. Device according to claim 1, **characterised in that** the first and second pair of connection contacts (35A, 35G; 35C, 35E) are arranged on a connection element (30E) of the cover (30).

3. Device according to claim 2, **characterised in that** the connection contacts (35A, 35G; 35C, 35E) are arranged so as to lie next to one another on the connection element (30E).

4. Device according to either claim 2 or claim 3, **characterised in that** the first conductor track (33) has a first portion (33A) and a second portion (33B), the two ends of the first portion and the two ends of the second portion of the first conductor track being arranged on the connection element (30E) of the cover (30) and
the second conductor track (34) having a first portion (34B) and a second portion (34A), the two ends of the first portion and the two ends of the second portion of the second conductor track being arranged on the connection element (30E) of the cover (30).

5. Device according to claim 4, **characterised in that** one end of the first portion of the first conductor track and one end of the first portion of the second conductor track form the connection contacts (35A, 35G) of the first pair of connection contacts and
one end of the second portion of the first conductor track and one end of the second portion of the second conductor track form the connection contacts (35C, 35E) of the second pair of connection contacts.

6. Device according to claim 5, **characterised in that** the other end of the first portion of the first conductor track and the other end of the second portion of the first conductor track form a third pair (35H, 35F) of connection contacts and the other end of the first portion of the second conductor track and the other end of the second portion of the second conductor track form a fourth pair (35B, 35D) of connection contacts.

7. Device according to any of claims 1 to 6, **characterised in that** the cover (30) has one or more cutouts (39A, 39B).

8. Device according to any of claims 1 to 7, **characterised in that** the cover (30) is U-shaped.

9. Device according to any of claims 1 to 8, **characterised in that** the base material (31) is a non-woven material.

10. Device according to any of claims 1 to 9, **characterised in that** the conductor tracks (33, 34) are printed on the base material (31) by means of a screen printing method.

11. Device (B) for monitoring an access to a patient for an apparatus with which a fluid is supplied to a patient and/or a fluid is carried away from the patient via a tube, comprising a device for detecting moisture according to any of claims 1 to 10.

12. Device according to claim 11, **characterised in that** the monitoring device (B) has an evaluation unit (41) which can be connected to the device (40) for detecting moisture.

13. Device according to either claim 11 or claim 12, **characterised in that** the monitoring device (B) has a connection part (50) to which the device (40) for detecting moisture can be connected.

14. Device according to claim 13, **characterised in that** the connection part (50) has a first and second pair (53A, 53G; 53C, 53E) of connection contacts for contacting the first and second pair (35A, 35G; 35C, 35E) of connection contacts of the device (40) for detecting moisture, the first pair (53A, 53G) of connection contacts being connected to a connection cable (42) for electrically connecting the evaluation unit (41) of the monitoring device (B) to the device (40) for detecting moisture, and the second pair (53C, 53E) of connection contacts being electrically interconnected by means of a terminating resistor (R).

15. Device according to claim 14, **characterised in that** the terminating resistor (R) is an SMD resistor.

16. Device according to either claim 14 or claim 15, **characterised in that** the connection part (50) of the monitoring device (B) has four further connection contacts (53B, 53D; 53F, 53H), of which two connection contacts are electrically interconnected in each case.

17. Device according to any of claims 13 to 16, **characterised in that** the connection part (50) of the monitoring device (B) is designed as a clamping device for clamping the cover (30).

18. Blood treatment device comprising an extracorporeal blood circuit (I) which has an arterial blood line (6) having an arterial cannula (5) and a venous blood line (7) having a venous cannula (8), and comprising a device (B) for monitoring the arterial and/or venous vascular access according to any of claims 11 to 17.

## Revendications

1. Dispositif servant à détecter l'humidité, destiné à être utilisé avec un dispositif servant à surveiller un accès à un patient pour un appareil, à l'aide duquel un liquide est amené à un patient et/ou un liquide est prélevé du patient par l'intermédiaire d'une conduite flexible, le dispositif servant à détecter l'humidité étant réalisé sous la forme d'un patch (30) à poser sur la peau du patient, lequel présente un matériau de support (31) flexible, sur lequel est rapportée, sous la forme d'un capteur d'humidité, une structure (32) électroconductrice composée de pistes conductrices (33, 34), laquelle structure présente une première piste conductrice (33) et une deuxième piste conductrice (34), une des extrémités de la première piste conductrice et une des extrémités de la deuxième piste conductrice présentant une première paire (35A, 35G) de contacts de raccordement rapportés sur le matériau de support (31) aux fins du raccordement du dispositif servant à surveiller l'accès vasculaire,
**caractérisé en ce que** l'autre extrémité de la première piste conductrice (33) et l'autre extrémité de la deuxième piste conductrice (34) présente une deuxième paire (35C, 35E) de contacts de raccordement rapportés sur le matériau de support (31) aux fins du raccordement d'une résistance de terminaison, qui ne fait pas partie intégrante du dispositif servant à détecter l'humidité.

2. Dispositif selon la revendication 1, **caractérisé en ce que** la première et la deuxième paire de contacts de raccordement (35A, 35G ; 35C, 35E) sont disposées au niveau d'un élément de raccordement (30E) du patch (30).

3. Dispositif selon la revendication 2, **caractérisé en ce que** les contacts de raccordement (35A, 35G ; 35C, 35E) sont disposés les uns à côté des autres au niveau de l'élément de raccordement (30E).

4. Dispositif selon la revendication 2 ou 3, **caractérisé en ce que** la première piste conductrice (33) présente une première portion (33A) et une deuxième portion (33B), les deux extrémités de la première portion et les deux extrémités de la deuxième portion de la première piste conductrice étant disposées au niveau de l'élément de raccordement (30E) du patch (30), et
**en ce que** la deuxième piste conductrice (34) présente une première portion (34B) et une deuxième portion (34A), les deux extrémités de la première portion et les deux extrémités de la deuxième portion de la deuxième piste conductrice étant disposées au niveau de l'élément de raccordement (30E) du patch (30).

5. Dispositif selon la revendication 4, **caractérisé en ce qu'**une des extrémités de la première portion de la première piste conductrice et une des extrémités de la première portion de la deuxième piste conductrice forment les contacts de raccordement (35A, 35G) de la première paire de contacts de raccordement, et
**en ce qu'**une des extrémités de la deuxième portion de la première piste conductrice et une des extrémités de la deuxième portion de la deuxième piste conductrice forment les contacts de raccordement (35C, 35E) de la deuxième paire de contacts de raccordement.

6. Dispositif selon la revendication 5, **caractérisé en ce que** l'autre extrémité de la première portion de la première piste conductrice et l'autre extrémité de la deuxième portion de la première piste conductrice forment une troisième paire (35H, 35F) de contacts de raccordement, et
**en ce que** l'autre extrémité de la première portion de la deuxième piste conductrice et l'autre extrémité de la deuxième portion de la deuxième piste conductrice forment une quatrième paire (35B, 35D) de contacts de raccordement.

7. Dispositif selon l'une quelconque des revendications 1 à 6, **caractérisé en ce que** le patch (30) présente une ou plusieurs découpes (39A, 39B).

8. Dispositif selon l'une quelconque des revendications 1 à 7, **caractérisé en ce que** le patch (30) est réalisé de manière à présenter une forme de U.

9. Dispositif selon l'une quelconque des revendications 1 à 8, **caractérisé en ce que** le matériau de support (3) est un non-tissé.

10. Dispositif selon l'une quelconque des revendications 1 à 9, **caractérisé en ce que** les pistes conductrices (33, 34) sont imprimées sur le matériau de support (31) par un procédé de sérigraphie.

11. Dispositif (B) servant à surveiller un accès à un patient destiné à un équipement, à l'aide duquel un liquide est amené à un patient et/ou un liquide est prélevé du patient par l'intermédiaire d'une conduite flexible, comprenant un dispositif servant à détecter l'humidité selon l'une quelconque des revendications 1 à 10.

12. Dispositif selon la revendication 11, **caractérisé en ce que** le dispositif de surveillance (B) présente une unité d'analyse (41) pouvant être raccordée au dispositif servant à détecter l'humidité (40).

13. Dispositif selon la revendication 11 ou 12, **caractérisé en ce que** le dispositif de surveillance (B) présente une partie de raccordement (50), à laquelle le dispositif (40) servant à détecter l'humidité peut être raccordé.

14. Dispositif selon la revendication 13, **caractérisé en ce que** la partie de raccordement (50) présente une première et une deuxième paire (53A, 53G ; 53C, 53E) de contacts de raccordement servant à établir un contact avec la première et la deuxième paire (35A, 35G ; 35C, 35E) de contacts de raccordement du dispositif (40) servant à détecter l'humidité, les contacts de raccordement de la première paire (53A, 53G) étant raccordés à un câble de liaison (42) servant à établir une liaison électrique entre l'unité d'analyse (41) du dispositif de surveillance (B) et le dispositif servant à détecter l'humidité (40) et les contacts de raccordement de la deuxième paire (53C, 53E) étant reliés entre eux de manière électrique par l'intermédiaire d'une résistance de terminaison (R).

15. Dispositif selon la revendication 14, **caractérisé en ce que** la résistance de terminaison (R) est une résistance SMD.

16. Dispositif selon la revendication 14 ou 15, **caractérisé en ce que** la partie de raccordement (50) du dispositif de surveillance (B) présente quatre autres contacts de raccordement (53B, 53D ; 53F, 53H), dont respectivement deux contacts de raccordement sont reliés entre eux de manière électrique.

17. Dispositif selon l'une quelconque des revendications 13 à 16, **caractérisé en ce que** la partie de raccordement (50) du dispositif de surveillance (B) est réalisée sous la forme d'un dispositif de serrage servant à coincer le patch (30).

18. Dispositif de traitement du sang comprenant un circuit sanguin extracorporel (I), qui présente une conduite de sang artérielle (6) pourvue d'une canule artérielle (5) et une conduite de sang veineuse (7) pourvue d'une canule veineuse (8), et comprenant un dispositif servant à surveiller (B) l'accès vasculaire artériel et/ou veineux selon l'une quelconque des revendications 11 à 17.
